# EUROPÄISCHE PATENTSCHRIFT

(11) **EP 0 509 303 B1**
(45) Veröffentlichungstag und Bekanntmachung des Hinweises auf die Patenterteilung: **25.01.1995**
(21) Anmeldenummer: 92105446.6
(22) Anmeldetag: 30.03.1992
(51) Int. Cl.: C07C 265/04, C07C 263/04, C07C 271/04

(54) **Verfahren zur Herstellung von beta-Halogen-tert.-alkylisocyanaten**
Method of production of beta-halo-t-alkylisocyanates
Procédé pour la production de beta-halo-t-alkylisocyanates

(30) Priorität: 12.04.1991 DE 4111904
(43) Veröffentlichungstag der Anmeldung: 21.10.1992
(73) Patentinhaber: BAYER AG, 51368 Leverkusen (DE)
(72) Erfinder: Schubart, Rüdiger, Dr., W-5060 Bergisch Gladbach (DE)

(56) Entgegenhaltungen:
- EP-A- 0 025 907
- EP-A- 0 037 481
- BE-A- 620 028
- CH-A- 425 768
- FR-A- 2 107 796
- SYNTHESIS Nr. 2, 1980, STUTTGART Seiten 85 - 110; V I GORBATENKO ET AL:'Synthesis and reactions of alpha-haloalkyl isocyanates'

## Beschreibung

Die Erfindung betrifft ein neues Verfahren zur Herstellung von bekannten β-Halogen-tert.-alkylisocyanaten, die als Zwischenprodukte für die Synthese von Kautschukhilfsmitteln oder von biologisch aktiven Verbindungen, wie beispielsweise Herbiziden (vgl. z.B. EP-A 294 666) eingesetzt werden können.

Es ist bereits bekannt, daß man β-Monochlor-tert.-alkylisocyanate erhält, wenn man β-Hydroxy-tert.-alkylamine zunächst mit Thionylchlorid in β-Chlor-tert.-alkylaminhydrochloride und diese anschließend mit Phosgen in die Isocyanate überführt (vgl. DE-OS 2 045 906).

Nachteilig an diesem Verfahren sind die schlechten Ausbeuten an den chlorierten tert.-Alkylisocyanaten.

Es ist ferner bereits bekannt, Methylisocyanat mit Chlor unter Belichtung umzusetzen (vgl. Houben-Weyl Band E4, Seite 1171). Auf diese Weise erhält man jedoch das mehrfach chlorierte Chlorcarbonyl-isocyaniddichlorid.

Desweiteren wird in der EP-A-0 025 907 ein Verfahren zur Synthese halogenierter tertiärer Alkylisocyanate beschrieben, indem man tertiäre Alkylisocyanate oder tertiäre Carbamidsäurehalogenide mit Halogen, Sulfonylchlorid und/oder Sulfurylbromid umsetzt.

Weiterhin ist aus der Literatur bekannt, daß HCl-Additionsprodukte von Isocyanaten zu Fragmentierungen neigen (vgl. Houben-Weyl Band E4, Seite 63).

Beispielsweise bewirkt die weitere Addition von Chlorwasserstoff an N-tert.-Butyl-N-vinylcarbamidsäurechlorid die Abspaltung des tert.-Butylrestes als tert.-Butylchlorid.

Folglich liegt die Schlußfolgerung nahe, daß die Chlorierung von tert.-Alkylcarbamidsäurechloriden ebenfalls zur Abspaltung von tert.-Alkylchloriden führen müßte. In der Literatur finden sich zudem Hinweise, daß bei Temperaturen um und über 200°C mit der Abspaltung der Chlorcarbonylgruppe als Phosgen zu rechnen ist [vgl. Angew. Chem. 74, 848 (1962)].

Es wurde nun gefunden, daß man β-Halogen-tert.-alkylisocyanate der Formel (I) 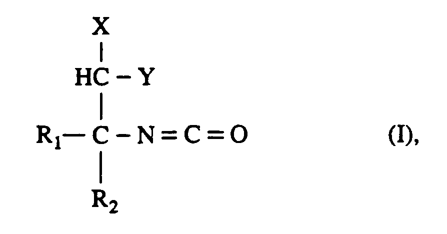
in welcher
- X: für Chlor steht,
- Y: für Wasserstoff oder Chlor steht,
- R₁: für jeweils geradkettiges oder verzweigtes Alkyl oder Halogenalkyl steht und
- R₂: für jeweils geradkettiges oder verzeigtes Alkyl, Halogenalkyl oder für gegebenenfalls durch Halogen und/oder Trifluormethyl substituiertes Phenyl steht,
in guten Ausbeuten und hoher Reinheit erhält, wenn man tert.-Alkylisocyanate der Formel (II) 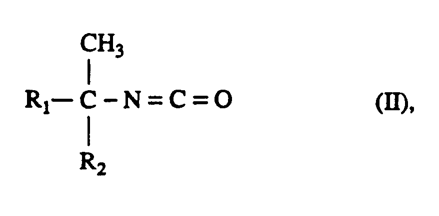
in welcher
R₁ und R₂ die oben angegebene Bedeutung haben,
zunächst durch Einleiten von HCl-Gas in die entsprechenden tert.-Alkylcarbamidsäurechloride überführt, anschließend diese mit elementarem Chlor, gegebenenfalls unter Belichtung, gegebenenfalls in Gegenwart von Katalysatoren und gegebenenfalls in geeigneten Lösungsmitteln in einer geeigneten, üblichen Laborapparatur bei Temperaturen zwischen -20°C und +50°C, bei Normaldruck oder leichtem Überdruck von bis zu 2.000 mbar, umsetzt. Die gewünschten Monochlor-tert.-alkylisocyanate erhält man schließlich durch Erhitzen der Reaktionslösung, wobei Chlorwasserstoff wieder freigesetzt wird.

Es ist ausgesprochen überraschend, daß diese Verfahrensweise der Chlorierung in guter Ausbeute die gewünschten β-Halogen-tert.-alkylisocyanate der Formel (I) ergibt. Aufgrund obengenannter Literaturstellen mußte nämlich mit Abspaltung der tert.-Alkylreste in Form von tert.-Alkylchlorid (vgl. Houben-Weyl Bd. E4, Seite 63) und insbesondere mit Fragmentierungen bei der Umsetzung von Isocyanaten bzw. deren HCl-Addukte, den Carbamidsäurechloriden, gerechnet werden (vgl. Houben-Weyl 4/56 Photochemie I und II, 891-892).

Mit Hilfe des erfindungsgemäßen Verfahrens erhält man bevorzugt Verbindungen der Formel (I), in welcher R₁ für Methyl, Ethyl, n- oder iso-Propyl, n-, iso-, sec.- oder tert.-Butyl steht, R₂ für Methyl, Ethyl, n- oder iso-Propyl, n-, iso-, sec.- oder tert.-Butyl oder für gegebenenfalls einfach bis dreifach, gleich oder verschieden, durch Fluor, Chlor, Brom und/oder Trifluormethyl substituiertes Phenyl steht, besonders bevorzugt stehen R₁ für Methyl oder Ethyl und R₂ für Methyl, Ethyl oder Phenyl, insbesondere für Methyl.

Ganz besonders bevorzugt lassen sich nach dem erfindungsgemäßen Verfahren die Verbindungen der Formel (I) herstellen, in denen X für Chlor und Y für Wasserstoff stehen, d.h. β-monochlorierte tert.-Alkylisocyanate.

Das erfindungsgemäße Verfahren läßt sich bei Verwendung von tert.-Butylisocyanat durch folgendes Formelschema darstellen: 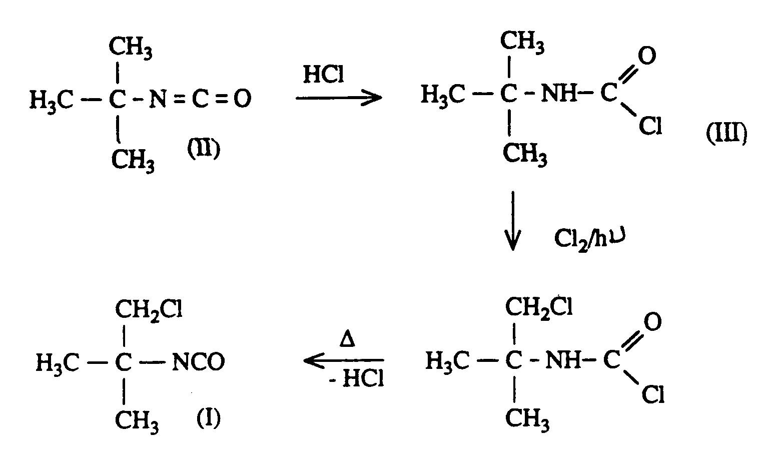
Das erfindungsgemäße Verfahren kann als "Eintopfreaktion" ohne, aber auch in Gegenwart von Verdünnungsmitteln durchgeführt werden. Man kann alle für solche Halogenierungsreaktionen üblichen Verdünnungsmittel wie beispielsweise Dichlormethan, Chloroform oder Tetrachlormethan einsetzen. Bevorzugt verwendet man Tetrachlormethan.

Vorzugsweise wird das erfindungsgemäße Verfahren unter Zusatz von Verdünnungsmitteln durchgeführt. Das erfindungsgemäße Verfahren kann gegebenenfalls unter Belichtung oder durch Zugabe geeigneter Katalysatoren durchgeführt werden.

Die Bestrahlung wird beispielsweise mit einem wassergekühlten Quecksilber-Hochdruckbrenner durchgeführt, wobei die Halogenierungslampe sowohl als Tauchlampe eingesetzt, als auch von außen angebracht werden kann. Bei der Anbringung der Lampe ist darauf zu achten, daß möglichst viel Licht in die Halogenierungszone gelangt. Alle für solche Halogenierungen üblichen Quecksilber-Hochdruckbrenner lassen sich für das erfindungsgemäße Verfahren einsetzen. Natürlich sind auch andere für solche Halogenierungen geeignete Lampen einsetzbar.

Als Katalysatoren verwendet man vorzugsweie Peroxide, wie beispielsweise Cumylperoxid oder Benzoylperoxid in den üblichen Konzentrationen.

Die Reaktionstemperaturen können bei der Durchfürung des erfindungsgemäßen Verfahrens in einem größeren Bereich variiert werden. Im allgemeinen arbeitet man bei Temperaturen zwischen -20°C und 60°C, bevorzugt zwischen 0°C und 50°C, besonders bevorzugt bei Raumtemperaturen.

Das erfindungsgemäße Verfahren kann bei Normaldruck oder bei Überdruck bis zu 2.000 mbar durchgeführt werden. Im allgemeinen arbeitet man bei Normaldruck.

Zur Durchführung des Verfahrens leitet man Chlorwasserstoffgas in eine Mischung von tert.-Alkylisocyanat und Tetrachlormethan in einer üblichen Laborapparatur bis zur Sättigung ein.

Anschließend setzt man für den Fall, daß die monohalogenierten Verbindungen der Formel (I) erhalten werden sollen (d.h. X = Chlor und Y = Wasserstoff) pro Mol tert.-Alkylisocyanat der Formel (II), bzw. nach HCl-Addition tert.-Alkylcarbamidsäurechlorid der Formel (III), im allgemeinen stöchiometrische Mengen, oder einen Überschuß, bevorzugt bis 1,2 Mol bzw. besonders bevorzugt 1,1 Mol an Chlor ein.

Falls das Verfahren in Gegenwart von Katalysatoren durchgeführt wird, setzt man pro Mol tert.-Alkylisocyanat der Formel (II), bzw. tert.-Alkylcarbamidsäurechlorid der Formel (III), im allgemeinen 0,001 bis 1,5 Mol%, bevorzugt 0,02 bis 1 Mol% und besonders bevorzugt 0,1 bis 0,5 Mol% an Katalysator zu.

Dieses Verfahren der Sumpfhalogenierung, nach vorangegangener Hydrohalogenierung, kann als "Eintopfverfahren" bezeichnet werden und wird im allgemeinen bis zu einem Umsatz von 40 bis 80 %, bevorzugt 45 bis 65 %, besonders bevorzugt 50 bis 55 %, durchgeführt.

Der Vorteil dieser Verfahrensweise liegt darin, daß der Anteil der Nebenprodukte und damit die Menge des Materialverlustes (d.h. Verlust an Edukt der Formel (II)) deutlich geringer wird.

Auf diese Weise laßt sich die Ausbeute an β-Halogen-tert.-alkylisocyanat aus einer bestimmten Menge tert.-Alkylisocyanat deutlich verbessern.

Dieses Verfahren erlaubt aber auch die Möglichkeit der Chlorierung eines Gemisches von tert.-Alkylisocyanat mit tert.-Alkylcarbamidsäurechlorid, wie es z.B. bei unvollständiger Hydrochlorierung von tert.-Alkylisocyanat in der ersten Stufe auftreten kann, oder wenn die Chlorierung des tert.-Alkylisocyanats ohne vorherige Hydrohalogenierung begonnen wird und der entstandene Chlorwasserstoff erst zur Bildung des tert.-Carbamidsäurechlorids führt.

### Beispiel

500 g (∼5 Mol) tert.-Butylisocyanat werden in 500 ml Tetrachlormethan bei ungefähr 10°C mit Chlorwasserstoffgas gesättigt Anschließend leitet man Chlorgas in die Reaktionslösung und chlorierte bei 20 bis 32°C unter Belichtung mit einem Quecksilber-Hochdruckbrenner, der seinerseits ins Reaktionsmedium eintaucht und mit Wasser gekühlt wird. Die Zusammensetzung des Chlorierungsgemisches ist unmittelbar nach beendeter Chlorierung folgende (in Flächen %), wobei die Bestimmung indirekt gaschromatographisch über den jeweiligen Anteil des entsprechenden Isocyanats erfolgt: 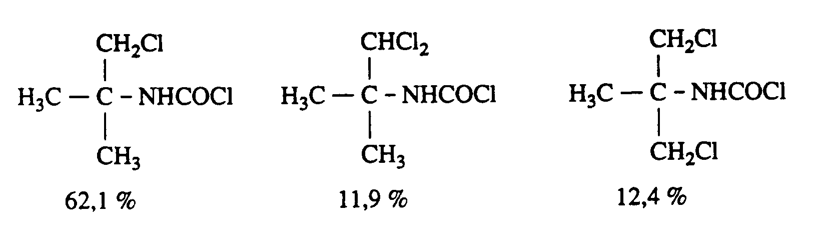
Daneben liegen noch 8 % Edukt und 5,6 % unbekannte Nebenprodukte vor.

Nach Beendigung des Chlorierungsvorgangs erwärmt man das Reaktionsgemisch. Dies führt zum Austreiben noch gelösten Chlorwasserstoffs, aber auch zur Dehydrohalogenierung der gelösten tert.-Butylcarbamidsäurechloride. Schließlich destilliert man das Lösungsmittel Tetrachlormethan ab, wobei man bis zu einer Temperatur von 120°C erhitzt. Hier unterbricht man den Vorgang, laßt abkühlen und erwärmt nochmals, diesmal unter Vakuum.

Durch Feinfraktionierung erhält man bei einem Siedepunkt von 50°C und 27,7 mbar 383 g Monochlor-tert.-butylisocyanat (60 % der Theorie), wobei zurückgewonnenes Edukt erneut eingesetzt wird.

Im Sumpf liegen noch die beiden isomeren Dichlor-tert.-butylisocyanate vor, die ebenfalls destillativ getrennt werden können.

## Patentansprüche

1. Verfahren zur Herstellung von β-Halogen-tert.-alkylisocyanate der Formel (I) 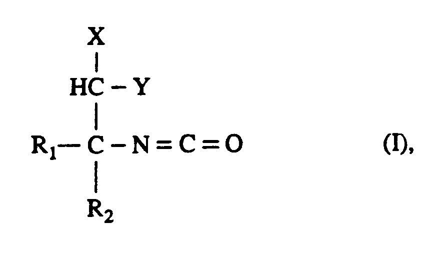 in welcher
X für Chlor steht,
Y für Wasserstoff oder Chlor steht,
R₁ für jeweils geradkettiges oder verzweigtes Alkyl oder Halogenalkyl steht und
R₂ für jeweils geradkettiges oder verzweigtes Alkyl, Halogenalkyl oder für gegebenenfalls durch Halogen und/oder Trifluormethyl substituiertes Phenyl steht,
dadurch gekennzeichnet, daß man tert.-Alkylisocyanate der Formel (II) 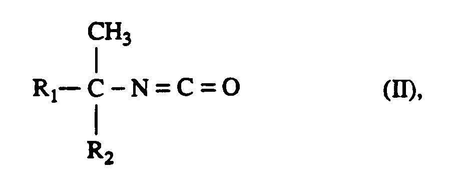 in welcher
R₁ und R₂ die oben angegebene Bedeutung haben,
mit Chlorwasserstoffgas in die entsprechenden tert.-Alkylcarbanddsäurechloride überführt, diese mit elementarem Chlor unter Belichtung oder in Gegenwart von Katalysatoren in einer geeigneten Apparatur chloriert und schließlich die β-Halogen-tert.-alkylisocyanate durch Dehydrohalogenierung bei erhöhter Temperatur freisetzt.

2. Verfahren gemäß Anspruch 1, dadurch gekennzeichnet, daß R₁ für Methyl, Ethyl, n- oder iso-Propyl, n-, iso-, sec.-, tert.-Butyl und deren monohalogenierte Derivate steht und R₂ für Methyl, Ethyl, n- oder iso-Propyl, n-, iso-, sec.- oder tert-Butyl und deren monohalogenierte Derivate oder für gegebenenfalls einfach bis dreifach, gleich oder verschieden durch Fluor, Chlor, Brom und/oder Trifluormethyl substituiertes Phenyl steht.

3. Verfahren gemäß Anspruch 1, dadurch gekennzeichnet, daß R₁ für Methyl oder Ethyl und R₂ für Methyl, Ethyl oder Phenyl stehen.

4. Verfahren gemäß Anspruch 1, dadurch gekennzeichnet, daß man Peroxide oder Azoverbindungen als Katalysatoren verwendet.

5. Verfahren gemaß Anspruch 1, dadurch gekennzeichnet, daß man die Umsetzung als Sumpfhalogenierung durchführt.

6. Verfahren gemaß Anspruch 5, dadurch gekennzeichnet, daß man die Reaktion bei einem Umsatz von 40 bis 80 % unterbricht.

7. Verfahren gemäß Ansprüchen 1 bis 6, dadurch gekennzeichnet, daß man den gesamten Prozeß als "Eintopfreaktion" durchführt.

## Claims

1. Process for the preparation of β-halogeno-tert.-alkyl isocyanates of the formula (I) 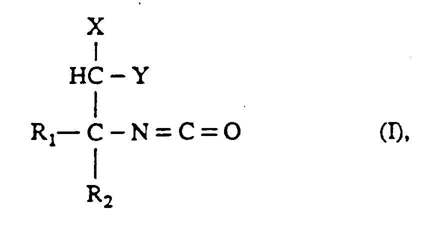 in which
X represents chlorine,
Y represents hydrogen or chlorine,
R₁ represents in each case straight-chain or branched alkyl or halogenoalkyl and
R₂ represents in each case straight-chain or branched alkyl, halogenoalkyl or optionally halogen- and/or trifluoromethyl-substituted phenyl,
characterized in that tert.-alkyl isocyanates of the formula (II) 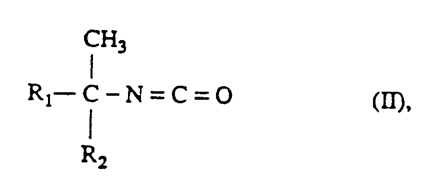 in which
R₁ and R₂ have the meaning given above,
are converted by hydrogen chloride gas to the corresponding tert.-alkylcarbamoyl chlorides, the latter are chlorinated with elemental chlorine under irradiation or in the presence of catalysts in a suitable apparatus and finally the β-halogeno-tert.-alkyl isocyanates are liberated by dehydrohalogenation at elevated temperature.

2. Process according to Claim 1, characterized in that R₁ represents methyl, ethyl, n- or iso-propyl, n-, iso-, sec.- or tert.-butyl and their monohalogenated derivatives, and R₂ represents methyl, ethyl, n- or iso-propyl, n-, iso-, sec.- or tert.-butyl and their monohalogenated derivatives or phenyl which is optionally mono- to trisubstituted by identical or different substituents from the series comprising fluorine, chlorine, bromine and/or trifluoromethyl.

3. Process according to Claim 1, characterized in that R₁ represents methyl or ethyl and R₂ represents methyl, ethyl or phenyl.

4. Process according to Claim 1, characterized in that peroxides or azo compounds are used as catalysts.

5. Process according to Claim 1, characterized in that the reaction is carried out as liquid phase halogenation.

6. Process according to Claim 5, characterized in that the reaction is interrupted at a conversion of 40 to 80%.

7. Process according to Claims 1 to 6, characterized in that the whole process is carried out as a "one-pot reaction".

## Revendications

1. Procède de production de β-halogéno-tertio-alkylisocyanates de formule (I) 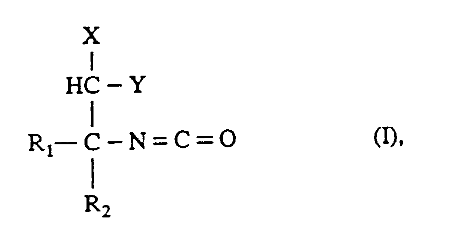 dans laquelle
X représente le chlore,
Y est de l'hydrogène ou du chlore,
R₁ est un groupe alkyle ou halogénalkyle linéaire ou ramifié et
R₂ est un groupe alkyle ou halogénalkyle linéaire ou ramifié ou un groupe phényle éventuellement substitué par un halogène et/ou un reste trifluorométhyle,
caractérisé en ce qu'on transforme des tertio-alkylisocyanates de formule (II) 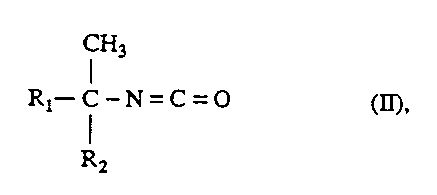 dans laquelle
R₁ et R₂ ont la définition indiquée ci-dessus,
avec du chlorure d'hydrogène gazeux en les chlorures d'acides tertio-alkylcarbamiques correspondants, on chlore ces derniers avec du chlore élémentaire dans un appareil approprié avec exposition à la lumière ou en présence de catalyseurs et on libère finalement les β-halogéno-tertio-alkylisocyanates par déshydrohalogénation à température élevée.

2. Procédé suivant la revendication 1, caractérisé en ce que R₁ est un groupe méthyle, éthyle, n-propyle, isopropyle, n-butyle, isobutyle, sec.-butyle, tertio-butyle et leurs dérivés mono-halogénés et R₂ est un groupe méthyle, éthyle, n-propyle, isopropyle, n-butyle, isobutyle, sec.-butyle, tertio-butyle et leurs dérivés mono-halogénés ou un groupe phényle portant éventuellement un à trois substituants fluoro, chloro, bromo et/ou trifluorométhyle identiques ou différents.

3. Procédé suivant la revendication 1, caractérisé en ce que R₁ est un groupe méthyle ou éthyle et R₂ est un groupe méthyle, éthyle ou phényle.

4. Procédé suivant la revendication 1, caractérisé en ce qu'on utilise comme catalyseurs des peroxydes ou des composés azoïques.

5. Procédé suivant la revendication 1, caractérisé en ce qu'on conduit la réaction comme halogénation en bas de colonne.

6. Procédé suivant la revendication 5, caractérisé en ce qu'on interrompt la réaction pour un degré de réaction de 40 à 80 %.

7. Procédé suivant les revendications 1 à 6, caractérisé en ce qu'on conduit la totalité des opérations comme "réaction en récipient unique".
